Europäisches Patentamt

European Patent Office

Office européen des brevets

(11) Publication number: **0 302 213**
**A1**

# EUROPEAN PATENT APPLICATION

(21) Application number: **88110175.2**

(22) Date of filing: **25.06.88**

(51) Int. Cl.⁴: **C07C 126/08**

(30) Priority: **04.08.87 CH 2980/87**

(43) Date of publication of application:
**08.02.89 Bulletin 89/06**

(84) Designated Contracting States:
**AT DE ES FR GB GR IT NL**

(71) Applicant: **AMMONIA CASALE S.A.**
**Via della Posta 4**
**CH-6900 Lugano(CH)**

Applicant: **Zardi, Umberto**
**Via Lucino 57**
**CH-6932 Breganzona (Ti)(CH)**

(72) Inventor: **Zardi, Umberto**
**Via Lucino 57**
**CH-6932 Breganzona(CH)**

(74) Representative: **Incollingo, Italo**
**AMMONIA CASALE S.A. Via della Posta 4**
**CH-6900 Lugano(CH)**

(54) **Process for the recovery of urea entrained by vapours in vacuum concentration systems.**

(57) In a process for the final concentration of urea, consisting by preference of two vacuum evaporation stages operating in series in which the vapour produced containing residual NH3 and CO2 and urea entrainments is separated in separators, preferably by gravity, to be condensated in systems to maintain total vacuum (vacuum systems) consisting by preference of surface condensers and steam ejectors, the product of said condensation (release condensate) being treated by preference by hydrolizing the urea and distilling the NH3 and the CO2 from the condensate in a final treatment system, the improvement being characterized by the fact that the vapour containing NH3 and CO2 and urea entrainments coming from the separators in the vacuum evaporation stages is brought into intimate contact with an aqueous urea solution with the ensuing passage of the urea entrained by the vapour to the solution itself before said vapour is sent to the vacuum system.

FIG. 1

# PROCESS FOR THE RECOVERY OF UREA ENTRAINED BY VAPOURS IN VACUUM CONCENTRATION SYSTEMS

In all processes for the production of urea there is a final concentration section for the elimination of the water produced in the synthesis section and introduced into the downstream sections and contained in the urea solution before concentration, in order to obtain melted urea which is practically anhydrous and then, by cooling, the finished granular product.

Said final concentration section usually consists in two hard vacuum evaporation stages, operating respectively with 70-75% vacuum (First stage) and 95-98 vacuum (Second stage), each of these stages comprising a heat exchanger and a separator for the vapours produced (mainly aqueous vapour) by the urea, each separator being connected to a system of condensation and extraction of inerts in two or more stages (vacuum system with vapour ejectors).

Even in the most advanced processes a considerable amount of urea is entrained by the vapours separated in the separators found in the two vacuum evaporation stages, such urea being therefore contained in the condensate extracted by the hydraulic guides in the vacuum systems.

Said condensate (discharge water) must therefore be treated in complex and expensive treatment plants where the urea content is hydrolyzed and the NH3 and CO2 so formed are separated by distillation together with the amounts of the same substances contained in the condensate. The NH3 and CO2 vapours thus obtained are then sent to the synthesis and recycle sections of the urea production plant to be transformed again into the finished product (urea).

These complex and expensive treatment plants make it thus possible to contain the consumption of raw materials (NH3 and CO2) and to keep to a tolerable level the pollution load in the waste water.

The size of these treatment plants for waste water depends essentially from the amount of urea to be treated and the urea production sections (recycle-synthesis) must also be oversize since they must be able to re-treat the gas produced by hydrolyzing the urea in the water-treatment section.

All this involves high investment costs and consumption of valuable energy (medium-pressure vapour) both in the section where the water is treated (hydrolyzer) and in the synthesis and recycle sections (distillation of the urea solution).

Besides, the urea entrained by the vapours in the vacuum separators causes the untimely fouling of the exchangers and the clogging of the vacuum section ejectors, resulting in unsatisfactory operational conditions (diminished vacuum). To bring the plant back to normal operating conditions often requires stopping the plant, thus incurring loss of production.

The separation of the urea entrained by the vapour in the vacuum separators, thus limiting the entrainment to a negligible fraction of product volume, could be achieved by using very complex separators (cyclone system, using demisters) with a high pressure drop, but this solution cannot be adopted owing to the fact that complex systems get easily clogged and that the pressure drop results in high energy consumption.

Large gravity separators are the system most commonly used from the above-mentioned urea concentrators, where an entrainment of 1 to 3% of product volume can be tolerated, with the ensuing troubles described above.

The object of this invention is a process for recovering economically the urea entrained by the vapours in the separators of the urea vacuum concentration sections, which makes it possible to avoid the troubles described above (high investment costs and energy consumption, loss of production).

With the process which forms the object of this invention, in the urea final concentration section, which generally consists of two stages of evaporation and separation of the vapours produced operating under vacuum, the vapours produced, separated from the urea in separators which generally work by gravity, and containing entrainments of urea equal to 1 to 3% of product volume, come into intimate contact with an aqueous urea solution which retains almost all the urea entrained by the vapours, before the latter are sent to the vacuum systems, thus reducing their urea content to less than 200 ppm (elimination of 99% of urea entrainments).

The aqueous urea solution enriched by the entrained urea, having reached the degree of concentration optimal for the system is recirculated to the final vacuum concentration section where it can be transformed into the finished product at the expense of a modest amount of low-pressure vapour.

The practically urea-free vapours are then treated without the drawbacks described above according to the known art in the vacuum sections and the waste water so obtained is much less polluted with urea and requires only limited treatment (reduction or elimination of the hydrolysis section).

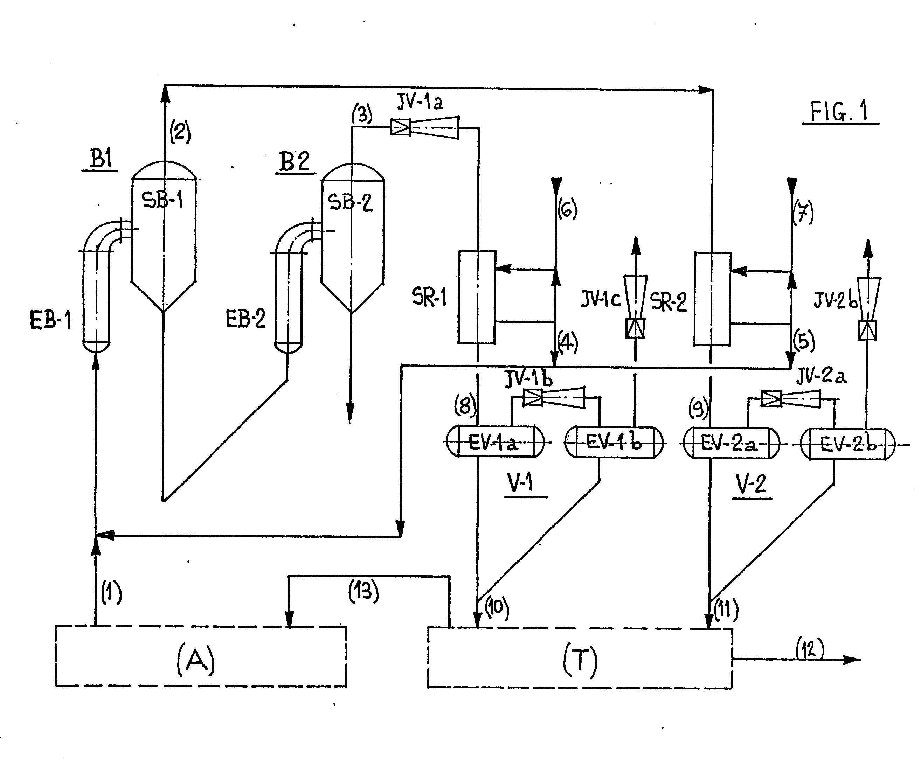

A description of the invention is supplied with reference to the drawing in Fig. 1, illustrating one of the

EP 0 302 213 A1

possible embodiments.

The urea solution (stream 1), produced according to the known art in section A (synthesis and recycle) is sent to vacuum concentration stages B1 and B2, also operating according to the known art, each of the stages consisting of an exchanger EB1 (respectively EB2) and of a gravity separator SB1 (respectively SB2) where almost the total amount of water contained in stream 1 is evaporated and separated.

The vapours (water with traces of NH3 and CO2) containing entrained urea (stream 2 from section B1 and stream 3 from section B2) are now, according to this invention, treated in recovery sections SR1 (respectively SR2) where almost the total amount of urea entrainment is eliminated owing to the intimate contact between the vapours and an aqueous urea solution, carried out in the section itself, with a negligible pressure drop in the vapours.

The urea recovered as an aqueous solution (stream 4, respectively 5) in the recovery sections SR1 and SR2 is then recycled directly to vacuum concentration sections B1 and B2.

A controlled amount of vapour condensate (stream 6 and 7) is sent to sections SR1 and SR2 to maintain the water balance.

The vapours (water with traces of NH3 and CO2) now practically free from urea (streams 8 and 9) are then sent to the vacuum systems V1 (respectively V2) each consisting of condensers EV1a and EV1b (respectively EV2a and EV2b) and of vapour ejectors JV1a, JV1b, JV1c (respectively JV2a, JV2b). The ejector JV1a of the vacuum system V1 is usually introduced directly downstream of separator SB2 so that the maximum degree of vacuum is maintained in it.

The waste condensate coming from the two vacuum systems V1 and V2 (steam 10 respectively 11) containing residual NH3 and CO2 and practically free from urea is finally sent to treatment section T where, by means of hydrolysis of the urea and distillation of the NH3 and CO2, the water is washed within the limits set by the regulations on pollution and discharged from the plant (stream 12), while the NH3 and CO2 vapours produced (stream 13) are recycled to the synthesis and recycle section A in order to reduce the consumption of raw materials.

According to a preferred embodiment of the invention the contact between the vapour with entrained urea and the aqueous urea solution takes place cocurrently, from top to bottom, surprisingly achieving the passage of almost the entire amount of urea contained in the vapours to the urea solution in a very short time and with extremely low pressure drop.

According to a further characteristic of the invention the contact between the vapours and the urea solution co-current from top to bottom is achieved by nebulizing the urea solution in drops smaller than 1000 micron in a hollow column where the vapours are kept for a period between 1 and 10 seconds.


Example 1


Application of the invention in newly built 1000 t/d plants.

| Investment: | |
|---|---|
| 1. Synthesis and recycle section A | Saving in expenditure by adopting the invention<br>- 250'000 Sfr. |
| 2. Final urea condensation section B1-B2 with vacuum systems V1-V2 | Greater expenditure by adopting the invention<br>+ 100'000 Sfr. |
| 3. Urea recovery section SR1 and SR2 | Greater expenditure by adopting the invention<br>+ 400'000 Sfr. |
| 4. Waste water treatment section T | Saving in expenditure by adopting the invention<br>- 300'000 Sfr. |

The total investment cost remains practically the same in both cases.

3

| Consumption of valuable vapour (20÷30 bar): | |
|---|---|
| 1. Synthesis and recycle section A | Lower consumption of 10 kg/T urea |
| 2. Final urea condensation section B1-B2 with vacuum systems V1-V2 | ------ |
| 3. Urea recovery section SR1 and SR2 | ------ |
| 4. Waste water treatment section T | Lower consumption of 50 kg/T urea. |

A saving is achieved, therefore, in the consumption of valuable vapour amounting to 60 kg per Tonn of urea produced, equal to a reduction in operating costs amounting to 330'000x60x0.04 = Sfr. 792'000 per annum.

Example 2

Application of the invention to improve an existing 1000 Tonn urea/day plant.

| Investment: | | |
|---|---|---|
| 1. Synthesis and recycle section A<br>2. Final urea condensation section B1-B2 with vacuum systems V1-V2 | | ------<br>------ |
| 3. Urea recovery section SR1 and SR2 | | Greater expenditure by adopting the invention + 400'000 Sfr. |
| | Total investment | 400'000 Sfr. |

Consumption of valuable vapour (20÷30 bar):

As in example 1 the consumption of valuable vapour is less by 60 kg/Tonn urea equal to a saving in operating costs amounting to Sfr. 792'000.

The investment can therefore be amortized in less than a year.

Example 3

Reference is made to Fig. 1.

In a 1000 Tonn/day urea production plant adopting the invention streams (10) and (11) equal to 500 Tonn/day of condensate showed a residual urea content of 100 ppm equal to 5= kg urea/day, after treatment in vapour systems SR1 and SR2 (steams 2 and 3) containing initially altogether 10'000 kg of urea/day.

Streams (2) and (3) were treated in systems SR1 and SR2 each of which consisted of a hollow vertical cylindrical column 400 mm in diameter at the top of which the vapour was put in contact with a flow of 500 m3/day of urea solution at 30% concentration nebulized in drops with a 500 micron diameter, the two streams running down the column from top to bottom in 2.5 seconds, with a pressure drop equal to 5 mm of water column.

## Claims

1. In a process for the final concentration of urea, consisting by preference in two stages of hard vacuum evaporation operating in series, in which the vapours produced with residual contents of NH3 and CO2 and entrained urea are separated, preferably by gravity, to be condensed in vacuum systems consisting by preference of surface condensers and vapour ejectors, the product of said condensation being treated by preference by hydrolyzing the urea and by distilling the NH3 and CO2 from the condensate in a final treatment system, the improvement is characterized by the fact that the vapours containing NH3 and CO2 and entrained urea coming from the vacuum evaporation stages are brought into intimate contact with an aqueous urea solution and consequently the urea entrained by the vapours is transferred to the solution itself before the said vapours are sent to the vacuum systems.

2. Process according to claim 1, characterized by the fact that contact between the vapours with entrained urea and the aqueous urea solution takes place cocurrently from top to bottom.

3. Process according to claim 2, characterized by the fact that contact between the vapours and the urea solution cocurrent from top to bottom is achieved by nebulizing the urea solution in drops below 1000 micron in size.

4. Process according to claim 3, characterized by the fact that contact between the vapours and the urea solution takes place in a hollow column where the vapours remain for between 1 and 10 seconds.

FIG. 1

## DOCUMENTS CONSIDERED TO BE RELEVANT

EP 88110175.2

| Category | Citation of document with indication, where appropriate, of relevant passages | Relevant to claim | CLASSIFICATION OF THE APPLICATION (Int. Cl.4) |
|---|---|---|---|
| A | DE - B2 - 2 438 685 (INVENTA) <br> * Claim 1 * | 1 | C 07 C 126/08 |
| A | DD - A1 - 228 545 (VEB AGROCHEMIE) <br> * Claim 1 * | 1 | |
| A | DE - A1 - 3 002 460 (CF INDUSTRIES PIESTERITZ) <br> * Claim 1 * | 1 | |
| A | EP - A2 - 0 144 964 (UNIE VAN KUNSTMESTFABRIEKEN) <br> * Abstract * | 1 | |
| A | EP - A1 - 0 066 906 (UNIE VAN KUNSTMESTFABRIEKEN) <br> * Claim 1 * | 1 | **TECHNICAL FIELDS SEARCHED (Int. Cl.4)** <br><br> C 07 C 126/00 |

The present search report has been drawn up for all claims

| Place of search | Date of completion of the search | Examiner |
|---|---|---|
| VIENNA | 02-11-1988 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82